# EUROPEAN PATENT SPECIFICATION

(11) **EP 2 736 595 B1**
(45) Date of publication and mention of the grant of the patent: **09.09.2015**
(21) Application number: 12745796.8
(22) Date of filing: 16.07.2012
(51) Int. Cl.: A61N 5/06

(54) **HANDHOLDABLE LASER DEVICE FEATURING PULSING OF A CONTINUOUS WAVE LASER**
HANDGEFÜHRTE LASERVORRICHTUNG MIT GEPULSTER ABGABE EINES DAUERSTRICHLASERS
DISPOSITIF LASER POUVANT ÊTRE TENU À LA MAIN ET COMPORTANT UNE ÉMISSION D'IMPULSIONS DE LASER CONTINU

(30) Priority: 28.07.2011 US 201161512431 P
(43) Date of publication of application: 04.06.2014
(73) Proprietor: ILUMINAGE BEAUTY LTD, P.O.B. 14 (IL)
(72) Inventor: BARTOLONE, John, Brian, Trumbull, Connecticut 06611 (US); SHERWOOD, Duncan, Reynolds, Trumbull, Connecticut 06611 (US)
(74) Representative: ZBM Patents - Zea, Barlocci & Markvardsen
(86) International application number: PCT/EP2012/063922
(87) International publication number: WO 2013/014018

(56) References cited:
- WO-A1-2010/150175
- US-A- 6 149 645
- US-A1- 2005 045 189
- US-A1- 2006 247 609

## Description

### BACKGROUND OF THE INVENTION

### Field of the Invention

The invention concerns a cordless handholdable laser device coupling modulated and continuous wave lasers in a system that maintains a constant wavelength. The laser device is useful to treat wrinkles and hyperpigmentation.

### The Related Art

Devices based on light amplification by stimulated emission of radiation (laser) have revolutionized many areas of dermatological medicine and of cosmetics. Amongst skin conditions responsive to treatment are acne scars, rosacea, hyperpigmentation, unwanted hair and dermal rejuvenation. Ablative resurfacing has become a common method for cosmetic rejuvenation. Wrinkle reduction has been a particular objective of the phototherapy.

Advances in laser based devices and their use in skin treatment methods have been many during the last decade. Several publications have focused on safe arming of the device to avoid unintended exposures. US 2004/0167502 A1 (Weckwerth et al.) reports optical sensors for detecting engagement with a skin surface. The sensors are based upon multiple light emitting diodes, each having a unique wavelength band, and a broad-band photodetector to measure the remission of light at multiple wavelengths from a material being analyzed. US 2010/0082020 (Gong et al.) describes a medical laser having a capacitance sensor and an emission control device to ensure that a laser handpiece is in contact with skin prior to activation. The handpiece needs to stand perpendicular to the skin surface before any surgical operation begins.

Non-uniform laser radiation treatments are described in US 7 856 985 B2 (Mirkov et al.). An output beam from a Nd:YAG laser is coupled with a diffractvie lens array. Most electromagnetic radiation delivery devices for treatment of skin are relatively large pieces of equipment. Complexity in their basic engineering and mode of operation defeats miniaturization into a handheld device. For instance, US 2008/0082089 A1 (Jones et al.) describes a system including a first solid-state and a second solid-state laser. A respective first output beam is fed into the second device for generating excitation in a rare earth doped gain medium to produce a second output beam. The latter is used to treat skin. US 2007/0179481 A1 (Frangineas et al.) seeks to treat skin laxity with a plurality of pulses from a carbon dioxide laser. The system requires a housing to contain a scanning apparatus and a tip connected to a vacuum pump for exhausting smoke resulting from ablation.

Many of the reported ablative procedures require special cooling mechanisms. For instance, US 5 810 801 directs a beam of radiation to penetrate the dermal region below a wrinkle to injure collagen. A cooling system is then activated to prevent injury of the overlying epidermis. These cooling systems are often quite bulky.

Another problem with the state of the art, particularly with portable instruments, is in their effectiveness to emit sufficiently energetic doses of electromagnetic radiation. US 2011/0040358 A1 (Bean et al.) provides one solution describing a portable device which is eye safe operating between 1350-1600 nm to treat wounds and diseases. This is a battery operated system that need not directly contact tissue. A key part of the device is a lens constructed to have the laser beam converge to a focal point slightly above the tissue surface target.

US 6 149 645 fails to teach a prism splitting the beam into multiple beamlets, as set out in claim 1.

### SUMMARY OF THE INVENTION

A laser device for treating skin is provided which includes:
(i) a handholdable housing;
(ii) a continuous wave laser member arranged within the housing and emitting an output beam;
(iii) a user activated switch system including a power activating button for arming the device and a power setting button to fire a single first pulse of output beam and after a pause in succession a single second pulse of output beam, the second pulse being automatically programmed to fire after the first pulse and pause; and
(iv) a lens array for receiving the output beam and transmitting the beam through a prism splitting the beam into multiple beamlets, each beamlet targeting a specific spot on the skin, and wherein beamlets from the first and second pulses successively strike the spot reinforcing energy applied to the specific spot.

### BRIEF DESCRIPTION OF THE DRAWING

Further features, aspects and benefits of the present invention will become more readily apparent from consideration of the following drawing in which:
Fig. 1 is a front view of one embodiment of the invention;
Fig. 2 is a plan perspective view of the embodiment according to Fig. 1;
Fig. 3 is a cross sectional view of Fig. 1 taken perpendicular to that view;
Fig. 4 is a view of the internal mechanism separated from the housing of Fig. 1;
Fig. 5 is a semi-schematic view of a portion of Fig. 4 encompassing the laser and printed circuitboard; and
Fig. 6 is an electrical overview of circuits for the shown embodiment.

### DETAILED DESCRIPTION OF THE INVENTION

Continuous wave lasers depend upon a beam whose output power is constant over time. A pulsed or modulated mode is where optical power appears in pulses of some duration at some repetition rate.

We have found a way to effectively deliver twice the energy of the continuous wave laser in a device that is not "built" to deliver that level of power. A continuous wave laser has been manipulated to behave like a modulated or pulsed laser. The present system accomplishes this dual characteristic by firing a first pulse of an output beam, followed by a pause and then firing a second pulse of the output beam. The second pulse is automatically programmed to fire after the first pulse subsequent to the pause. Power striking the skin is then amplified through a lens array which is a prism that splits the output beam into multiple beamlets. Each of the beamlets targets a very small specific spot on the skin. By successive first and second pulses, the resultant beamlets from these pulses successively strike the same specific spot. This reinforces the amount of energy applied to those specific spots. Automatic first and second pulses fire sufficiently quickly that a user will not have been able to remove the laser device from an area on the skin to which it has been applied and that through application has automatically caused the double pulse to be generated. Typically, the time duration of a pulse may range from 10 to 120 milliseconds, preferably from 40 to 80 milliseconds, and optimally about 60 milliseconds.

Two control buttons are activatable from outside the housing. One is a power activating button 14 functioning to arm/power on the device. The other is a power setting button 16 functioning to control the power level. The term "button" is to be interpreted broadly. Although in the first embodiment, the buttons are square, these may in other embodiments be of a round or other geometrical shape. Also these buttons may be movable inward/outward from a surface of the housing, but in another embodiment may be a non-movable touch screen form of switch.

In conjunction with the power setting button, there is a light emitting diode (LED) 18 for indicating the setting of high or low power 18a and 18b.

With the present device, a user can select either single pulse or double pulse modes of operation. The selection is accomplished by a user pressing power setting button 16 in a pre-programmed cadence pattern. The low power setting 18b will generate a single pulse. The high power setting 18a will generate two sequential pulses. After the first or second pulse in the respective low or high setting mode, the laser device will not fire again until the user repeats the cadence pattern on the power setting button. This procedure achieves a safe arming and also delivers power in a highly efficient manner from a relatively small device.

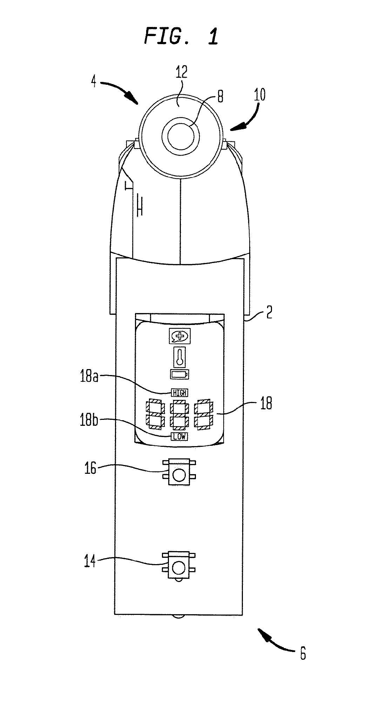

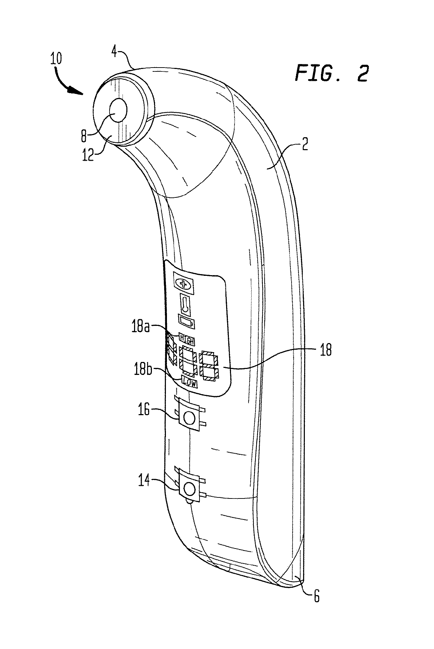

Fig. 1 and 2 reveal a first embodiment of this invention. The laser device features a curvilinear housing 2 having a first end 4 and an opposite second end 6. An aperture defining a window 8 is formed at a tip 10 of the first end of the housing.

The housing preferably has a sinusoidal or S-shape. This allows the tip 10 to be properly oriented against a user's face and simultaneously permits viewing by the user of power settings and activation. A longitudinal axis along a length of the housing and an axis traversing through the window at a point of intersection will define an angle between 100° and 170°, preferably between 110° and 160°, and optimally between 120° and 140°.

An annular plate 12 surrounds window 8. The plate is opaque to electromagnetic radiation. Any output beam of electromagnetic radiation is emitted through the window 8 which is an open central area of the annular plate.

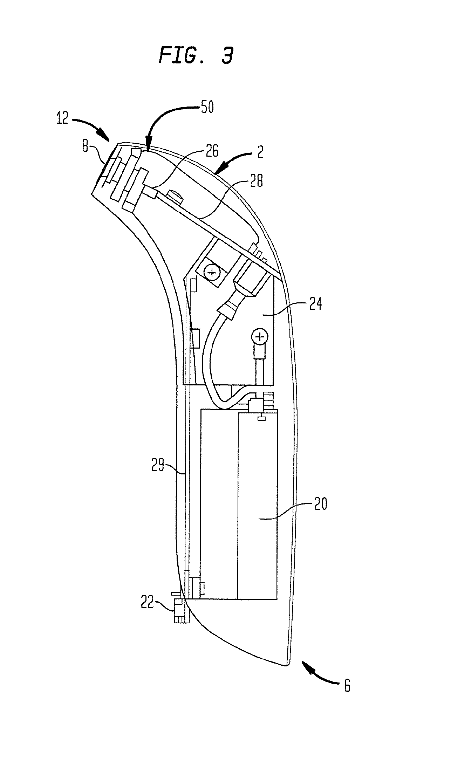

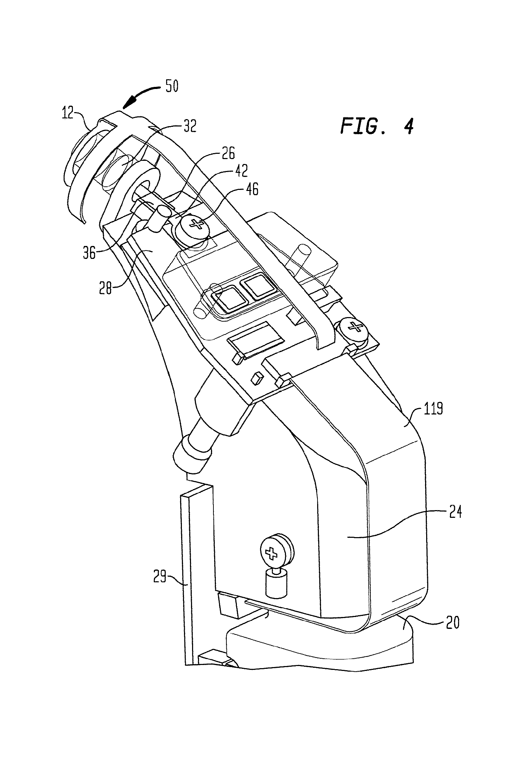

Fig. 3 and 4 reveal the inner mechanism of the laser device. A rechargeable battery 20 is lodged within a lower area of the housing just above the rear end 6. Recharging is achieved by connection of an outside power source to port 22 electrically communicating with the rechargeable battery.

Above the battery is an aluminum block 24 serving both as a support and solid coolant to dissipate heat generated by the laser member. The device neither needs nor features any special liquid or gas coolant system.

A laser member 26 generating electromagnetic radiation is supported on an arm of the aluminum block. The laser member of this embodiment operates on a constant output power delivering a continuous wave over time. It is a solid state diode laser including the elements indium, arsenic, gallium and tin. The laser produces a pulse of radiation having a wavelength between approximately 1300 and 1600 nm, preferably between 1420 and 1470 nm, and optimally about 1440 nm. Fluence may range between 0.5 and 5 joules/cm², more preferably between 1 and 3 joules/cm², and optimally between 1.3 and 1.8 joules/cm². Electromagnetic radiation emanating from the laser device is non-ablative to the skin being classified by the U.S. Food & Drug Administration as a Class I/1 inherently safe rating.

No lotions, creams or other chemicals need be applied to the skin target prior to the radiation treatment. The device of this invention needs no boost nor interacts with pre-positioned chemicals on the skin target area. Nonetheless, it may be desirable to cleanse the skin treatment area with a surfactant composition to avoid interference from makeup or other chemicals that might shield against the efficacy of applied electromagnetic radiation.

Downstream from the laser member arranged near the first end 4 is a diffractive lens array 32. An output beam of electromagnetic radiation from the laser member 26 is directed into the lens array which serves as a prism splitting the output beam into multiple beamlets. These beamlets constitute a larger diameter overall beam exiting the array and possess a non-uniform energy profile. Within the profile are a plurality of high-intensity zones surrounded by lower-intensity zones. Use of the defractive lens array allows the exiting beamlets to strike a broader area of the target skin. The higher-intensity zones heat selected portions of the target skin causing collagen shrinkage while the lower-intensity zones provide sufficient energy to stimulate collagen production. This combination allows a large area of the skin target to be treated simultaneously while minimizing the risk of burning or other damage to the skin.

Upstream from the laser member 26 is a printed circuitboard 28 supported on an arm of the aluminum block 24. Operation of the device is controlled by the printed circuitboard including power switching, radiation fire sequencing, generation, timing, sequencing of laser pulses and processing of skin contact information.

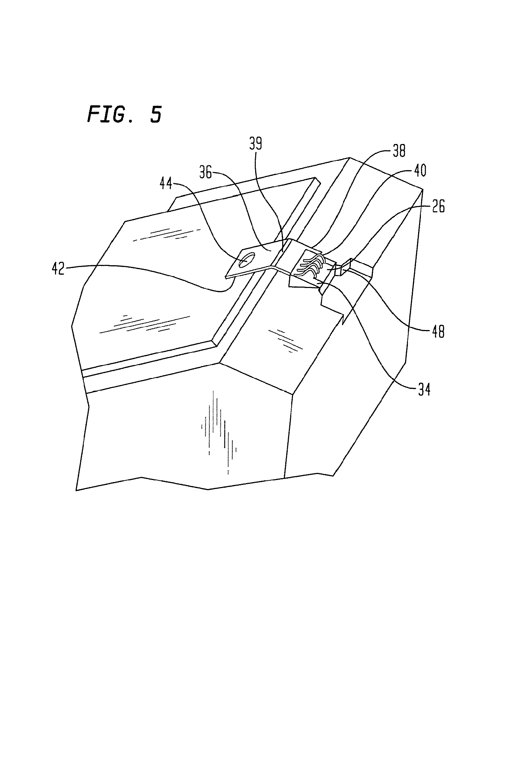

Between the laser member 26 and the aluminum block 24 is a submount 34 as best seen in Fig. 5. The submount directly supports the laser member and also a flexible electrically conductive connector 36 carrying signals/current from the printed circuitboard 28. The flexible electrically conductive connector features forward and rear ends 38, 42. An area 39 between the forward and rear ends is highly bendable. The bending may range from 0 to 360° in angle. This allows various angles between a major plane of the laser member and a major plane of the printed circuitboard. Preferably, the angle is held between 10 and 250°. This flexibility in orientation creates a geometric and ergonomic advantage.

The forward end of the flexible connector is bonded to the submount. A portion of the forward end features a set of several wire bonds 40 which complete the electrical connection to the laser member 26. The rear end 42 of the flexible connector features an aperture 44 for a screw 46 or other fastening member to achieve a press contact with the printed circuitboard. The screw and a washer assembly provides an evenly distributed force which compresses a large area of the flexible connector to a plated contact on the printed circuit board. This arrangement minimizes contact resistance, thus lowering electrical power loss. This arrangement also allows for ease of assembly, disassembly and replacement.

The flexibility of the connector allows the system to escape the ordinarily required connection of circuitry to be in a plane of the output beam generated by the laser. Flexible connectors in one embodiment of this invention are formed of a set of copper wires sandwiched between layers of polyimide.

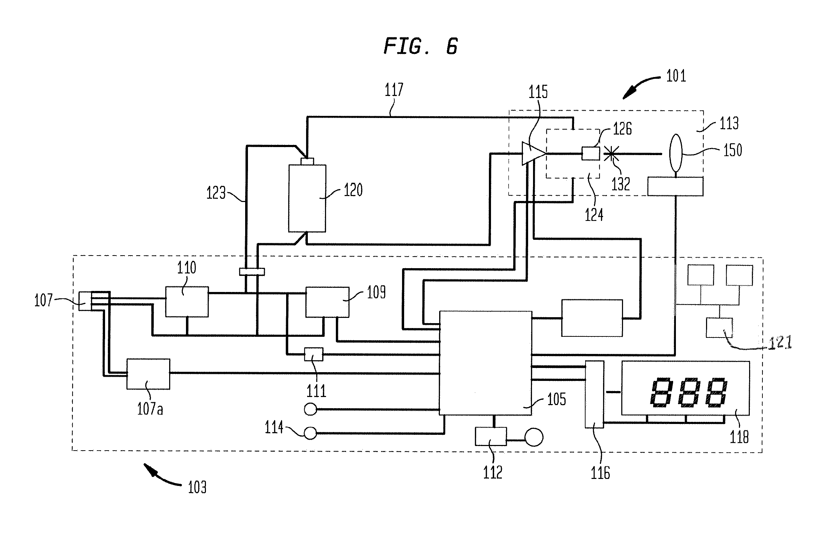

Fig. 6 reveals electrical relationships among elements constituting one embodiment of this invention. A problem with rechargeable battery operated devices is loss of power over time. The device of this invention may have an embodiment which is idled for long periods of time between uses. Consequently, it is necessary to draw as little power as possible from the battery 120 when in the sleep mode. This objective is achieved by having two power domains 101, 103. The first power domain 101 is controlled by a main circuit board 105 which draws very little current (around half a milliamp) when placed into a sleep mode. Wake up occurs when the main circuit board receives a signal generated either by the power button 114 or detection of a USB connectivity 107 via a USB serial communicator 107a. In sleep or shut down mode, voltage regulators 109, 111 which supply 3.3 and 5.0 volt power to the main circuit board are turned off.

Other features of the first power domain include a charger 110 for the battery, audio output 112, driver 116 for LED 118, and a real time clock 121. The real time clock is present for time stamping even when the laser device is shut down. An important feature of the real time clock is enforcement of a 24 hour delay between skin treatments. This mechanism is completely independent of the other safety control mechanisms that shut down operations on the main circuit board, and thereby serves as a double safety precaution against skin over exposure to laser radiation.

The second power domain 103 is controlled by a laser printed circuit board 113. Among components of board 113 is a laser enabling drive 115, laser member 126, aluminum block 124 (functioning as a heat sink), diffractive lens array 132 and contact (capacitive) sensor 150.

The battery 120 has two sets of leads. A first set of leads 117 connects directly to the laser printed circuit board 113 and heat sink 124. The first set of leads provides a high current and low resistance path for the current (around 30 amps) to run the laser member 126. Typically leads 117 may be an 16 gauge wire having DC resistance of 0.013 ohms per meter and an area of 1.3 mm². Lead wire for the first set may range in resistance from 0.004 to 0.06 ohms. Battery current flows from the positive high-current lead through the heat sink and the laser, exiting the cathode of the laser through a flexible circuit then reconnecting to the laser printed circuit board. A ribbon cable 119 connects the laser printed circuit board to the main circuit board. The battery is also charged through leads 117.

A second set of leads 123 connect battery 120 to the main circuit board 105. Wires for this connection are much thinner than those used in the first set of leads 117. Advantageously leads 117 relative to leads 123 have a relative gauge diameter ranging from 100:1 to 1.1:1, preferably from 50:1 to 1.1:1, and optimally from 3:1 to 1.5:1. For instance, leads 117 may be of 24 gauge wire with a DC resistance of 0.086 ohms per meter and a cross sectional area of 0.2 mm². Lead wire for the second set may range in resistance from 0.065 to 0.2 ohms. The second set of leads power control circuitry on the main circuit board and on the laser printed circuit board.

Use of two separate power connections to the battery avoids having to run any significant amount of power through the ribbon cable 119. This would be necessary were power only to come through the laser printed circuit board. The arrangement eliminates need for extra conductors in the ribbon cable and reduces electrical noise that might arise from extended wiring.

Benefit of having separate high power (laser) and low power (connected to circuit boards) is greater efficiency on space and improved safety because the circuit board must first be activated before the laser can be energized.

Fashioned in a downstream area of the submount 34 is an alignment structure 48 with outwardly tapering walls. The alignment structure receives the forward end 38 of the flexible connector to prevent movement and insuring the laser member is properly oriented.

A capacitive sensor electrode 50 is positioned at the first end 4 of the housing. An electrode terminates on each of three 120° sectors of the annular plate 12. A gap separates each of the sectors. The three electrodes are arranged in an annulus (representing a plane) to determine when a flat surface of suitable dielectric (i.e. skin) is sensed. By arranging the electrodes in a ring, they stay concentric to the cross section of the window 8 through which the electromagnetic radiation is emitted. The arrangement maximizes the surface area of the sensor and allows maintenance of the smallest possible volume around the window 8.

The capacitive sensor includes two conductors with a capacitance field between them. There are three capacitive switches related to each of the three electrodes. Each of the switches must satisfy a condition that it has the capacitance correlated with proper dry skin contact. When there is only partial contact with the skin, the dielectric is improper and firing of the laser cannot occur.

In summary, the present invention is described above in terms of a preferred and other embodiments. The invention is not limited, however, to the described and depicted embodiments. Rather, the invention is only limited by the claims appended hereto.

## Claims

1. A laser device for treating skin comprising:
(i) a handholdable housing (2);
(ii) a continuous wave laser member (26, 126) arranged within the housing and emitting an output beam;
(iii) a user activated switch system comprising a power activating button (14, 114) for arming the device and a power setting button (16) to fire a single first pulse of output beam and after a pause in succession a single second pulse of output beam, the second pulse being automatically programmed to fire after the first pulse and pause; and
(iv) a prism and a lens array (32, 132) for receiving the output beam and transmitting the beam through said prism (32, 132) splitting the beam into multiple beamlets, each beamlet targeting a specific spot on the skin, and wherein beamlets from the first and second pulses successively strike the spot reinforcing energy applied to the specific spot.

2. A device according to claim 1 wherein each of the first and second pulses have an identical time duration selected from a value within the range from 10 to 200 milliseconds.

3. A device according to claim 1 wherein each of the first and second pulses have an identical time duration selected from a value within the range from 40 to 80 milliseconds.

4. A device according to claim 1 wherein each of the first and second pulses have an identical time duration of about 60 milliseconds.

5. A device according to any one of the preceding claims wherein the output beam emits electromagnetic radiation of wavelength ranging from 1420 to 1470 nm.

6. A device according to any one of the preceding claims wherein the output beam has a fluence range from 0.5 to 5 joules/cm².

7. A device according to any one of the preceding claims wherein the continuous wave laser member is a solid state diode laser formed from elements selected from the group consisting of indium, arsenic, gallium, tin and combinations thereof.

8. A device according to claim 1 wherein the multiple beamlets form a larger diameter overall beam exiting the array than the diameter of the output beam, the overall beam possessing a non-uniform energy profile.

9. A device according to any one of the preceding claims which is powered by a battery (20, 120) within the housing, the battery having two sets of leads (117, 123), a first set providing power to the laser member (26, 126) and a second set providing power to a printed circuitboard (28, 105) controlling safety features.

## Patentansprüche

1. Ein Lasergerät zur Behandlung der Haut umfassend:
(i) ein handhaltbares Gehäuse (2);
(ii) ein Dauerstrichlaserelement (26, 126), das im Gehäuse angeordnet ist und einen Ausgangsstrahl ausstrahlt;
(iii) ein Benutzer-aktiviertes Schaltersystem umfassend einen Leistungsaktivierungsknopf (14, 114) zum Bereitmachen des Geräts und einen Leistungseinstellungsknopf (16) zum Erzeugen eines einzelnen ersten Ausgangsstrahlpulses und nach einer darauffolgenden Pause eines einzelnen zweiten Ausgangsstrahlpulses, wobei der zweite Puls automatisch programmiert ist, um nach dem ersten Puls und Pause erzeugt zu werden; und
(iv) ein Prisma und ein Linsenarray (32, 132) zum Empfangen des Ausgangsstrahls und zum Übertragen des Strahls durch das Prisma (32, 132), das den Strahl in mehrfache Teilstrahlen teilt, wobei jeder Teilstrahl auf eine bestimmte Stelle der Haut gezielt ist, und wobei Teilstrahlen der ersten und zweiten Pulse nacheinander die Stelle treffen, wodurch die auf die bestimmte Stelle angewandte Energie verstärkt wird.

2. Ein Gerät nach Anspruch 1, wobei jeder der ersten und zweiten Pulse eine identische Zeitdauer hat, die aus einem Wert im Bereich von 10 bis 200 Millisekunden ausgewählt ist.

3. Ein Gerät nach Anspruch 1, wobei jeder der ersten und zweiten Pulse eine identische Zeitdauer hat, die aus einem Wert im Bereich von 40 bis 80 Millisekunden ausgewählt ist.

4. Ein Gerät nach Anspruch 1, wobei jeder der ersten und zweiten Pulse eine identische Zeitdauer von etwa 60 Millisekunden hat.

5. Ein Gerät nach einem der vorhergehenden Ansprüche, wobei der Ausgangsstrahl elektromagnetische Strahlung einer Wellenlänge reichend von 1420 bis 1470 nm ausstrahlt.

6. Ein Gerät nach einem der vorhergehenden Ansprüche, wobei der Ausgangsstrahl einen Fluenzbereich von 0,5 bis 5 Joule/cm² hat.

7. Ein Gerät nach einem der vorhergehenden Ansprüche, wobei der Dauerstrichlaserelement ein Festkörperdiodenlaser ist, der aus Elementen besteht, die aus der Gruppe bestehend aus Indium, Arsen, Gallium, Zinn und Kombinationen davon ausgewählt sind.

8. Ein Gerät nach Anspruch 1, wobei die mehrfachen Teilstrahlen einen vom Array ausgehenden gesamten Strahl mit einem Durchmessers bilden, der größer als der Durchmesser des Ausgangsstrahls ist, wobei der gesamte Strahl ein ungleichmäßiges Energieprofil aufweist.

9. Ein Gerät nach einem der vorhergehenden Ansprüche, welches durch eine Batterie (20, 120) im Gehäuse angetrieben wird, wobei die Batterie zwei Reihen von Leitungen (117, 123) hat, wobei eine erste Reihe Leistung zum Laserelement (26, 126) bereitstellt und eine zweite Reihe Leistung zu einer Leiterplatte (28, 105) bereitstellt, die Sicherheitskenndaten kontrolliert.

## Revendications

1. Un dispositif laser pour le traitement de la peau comprenant :
(i) un boîtier (2) pouvant être tenu à la main ;
(ii) un élément laser à onde continue (26, 126) disposé dans le boîtier et émettant un faisceau de sortie ;
(iii) un système de commutation actionné par l'utilisateur comprenant un bouton d'activation de puissance (14, 114) pour armer le dispositif et un bouton d'ajustement de puissance (16) pour déclencher une première seule impulsion de faisceau de sortie et après une pause subséquente une deuxième seule impulsion de faisceau de sortie, dans lequel la deuxième impulsion est automatiquement programmée pour être déclenchée après la première impulsion et pause; et
(iv) un prisme et un ensemble de lentilles (32, 132) pour recevoir le faisceau de sortie et transmettre le faisceau au travers de ce prisme (32, 132) qui décompose le faisceau dans des multiples faisceaux partiels, étant adressé chaque faisceau partiel à un endroit spécifique de la peau, et dans lequel des faisceaux partiels des impulsions première et deuxième frappent successivement l'endroit, renforçant l'énergie appliquée sur l'endroit spécifique.

2. Un dispositif selon la revendication 1, dans lequel chacune des impulsions première et deuxième ont une durée identique choisie dans une valeur dans l'intervalle allant de 10 à 200 millisecondes.

3. Un dispositif selon la revendication 1, dans lequel chacune des impulsions première et deuxième ont une durée identique choisie dans une valeur dans l'intervalle allant de 40 à 80 millisecondes.

4. Un dispositif selon la revendication 1, dans lequel chacune des impulsions première et deuxième ont une durée identique d'environ 60 millisecondes.

5. Un dispositif selon l'une quelconque des revendications précédentes dans lequel le faisceau de sortie émet du rayonnement électromagnétique d'une longueur d'onde de 1420 à 1470 nm.

6. Un dispositif selon l'une quelconque des revendications précédentes dans lequel le faisceau de sortie a une gamme de fluence de 0,5 à 5 joules/cm².

7. Un dispositif selon l'une quelconque des revendications précédentes dans lequel l'élément laser à onde continue est une diode laser à l'état solide formée par des éléments choisis dans le groupe constitué de l'indium, de l'arsenic, du gallium, de l'étain et des combinaisons de ceux-ci.

8. Un dispositif selon la revendication 1 dans lequel les multiples faisceaux partiels forment un faisceau total sortant de l'ensemble qui a un diamètre plus grand que le diamètre du faisceau de sortie, ayant le faisceau total un profil énergétique non uniforme.

9. Un dispositif selon l'une quelconque des revendications précédentes qui est alimenté par une batterie (20, 120) dans le boîtier, ayant la batterie deux séries de lignes électriques (117, 123), une première série fournissant de la puissance à l'élément laser (26, 126) et une deuxième série fournissant de la puissance à un circuit imprimé (28, 105) contrôlant des caractéristiques de sécurité.
